# EUROPEAN PATENT SPECIFICATION

(11) **EP 2 733 484 B1**
(45) Date of publication and mention of the grant of the patent: **12.04.2017**
(21) Application number: 13005276.4
(22) Date of filing: 08.11.2013
(51) Int. Cl.: G01N 33/00, G01N 33/497

(54) **PORTABLE ELECTRONIC DEVICE WITH CHEMICAL SENSOR**
TRAGBARE ELEKTRONISCHE VORRICHTUNG MIT CHEMISCHEM SENSOR
DISPOSITIF ÉLECTRONIQUE PORTABLE AVEC UN CAPTEUR CHIMIQUE

(30) Priority: 14.11.2012 US 201213676770
(43) Date of publication of application: 21.05.2014
(73) Proprietor: Sensirion AG, 8712 Stäfa (CH)
(72) Inventor: Mayer, Felix, 8712 Stäfa (CH); Bürgi, Lukas, 8049 Zürich (CH)
(74) Representative: Mirza, Akram Karim

(56) References cited:
- WO-A1-2012/064252
- GB-A- 2 044 462
- US-A1- 2005 009 195
- US-A1- 2005 229 675
- R J HONICKY ET AL: "N-SMARTS: Networked Suite of Mobile Atmospheric Real-time Sensors", PROCEEDINGS OF THE 2008 SECOND ACM SIGCOMM WORKSHOP ON NETWORKED SYSTEMS FOR DEVELOPING REGIONS (NSDR), 18 August 2008 (2008-08-18), pages 1-5, XP055070322,
- ANAND D MANE ET AL: "Explosive detection with mobile telephony an attempt towards a safe ambience", SIGNAL PROCESSING, COMMUNICATION, COMPUTING AND NETWORKING TECHNOLOGIES (ICSCCN), 2011 INTERNATIONAL CONFERENCE ON, IEEE, 21 July 2011 (2011-07-21), pages 187-191, XP031940738, DOI: 10.1109/ICSCCN.2011.6024541 ISBN: 978-1-61284-654-5
- Michael Karst ET AL: "Humidity & Temperature Sensors in Mobile Phones", Wireless communication alliance event, 18 April 2012 (2012-04-18), pages 1-18, XP055069470, Retrieved from the Internet: URL:http://www.wca.org/event_archives/2012 /Sensirion_WCA_April2012_Mobile_Sensors.pd f [retrieved on 2013-07-03]

## Description

### FIELD OF THE INVENTION

The present invention relates to a portable electronic device such as a mobile phone, tablet and the like with an integrated chemical sensor being located within a duct through the exterior shell or housing of the device.

### BACKGROUND OF THE INVENTION

Portable or mobile devices originally introduced as mobile phones or electronic agendas become more and more ubiquitous. As the processing power of their internal processors grows and equally the bandwidth for communication with stationary processors, such portable devices take on more and more the role of multi-purpose tools available to consumers and specialist users alike.

It has been recognized that portable device can benefit from the presence of sensors capable of providing a chemical analysis of materials brought into contact or the vicinity of the device. Whilst there are many possible applications for such sensors, it suffices to consider for example the analysis of air surrounding the portable device. Such an analysis can be useful for multiple purposes such as testing for hazardous gases, breath analysis for general medical purposes or driving fitness, and the like.

However chemical sensors can often be rendered inefficient by lack of exchange of the medium to be analyzed, i.e. the analyte, within the immediate vicinity of the sensor. It is therefore seen as an object of the present invention to improve this exchange and hence prevent or reduce effects caused by saturation or stagnant flow.

"N-SMARTS: Networked Suite of Mobile Atmospheric Real-time Sensors", R.J. Honicky et al., Proceedings of the 2008 second ACM SIGCOMM Workshop on net worked systems for developing regions, August 18, 2008, pages 1-5, discloses the attaching of sensors to GPS enabled cell phones for gathering raw data necessary to begin to understand how urban air pollution impacts both individuals and communities. A hardware and software platform is introduced for exploring algorithms and data gathered from pollution sensors integrated into cell phones.

### SUMMARY OF THE INVENTION

Hence, according to a first aspect of the invention, there is provided a portable electronic device, preferably with telecommunication capabilities to allow for data and/or voice communication via private or public networks, enclosed in a housing having an air duct with an opening to the exterior of the housing and a chemical sensor with the duct connecting the chemical sensor to the outside and being linked with an actuator to move air along the duct and thus along a sensitive surface of the chemical sensor.

The portable device can be a smart phone, a handheld computer, a laptop, an electronic reader, a tablet computer, a game controller, a pointing device, a photo or a video camera, or a computer peripheral. Its housing is typically a shell of metal, glass, or plastic material and can be assembled as a unibody or from several parts. Enclosed in the housing are typically processors, drivers for parts such as screens, antennae, cameras, microphones and speakers as well as batteries to provide power to the device and its parts. A screen is typically arranged as a part of the housing or mounted behind a transparent window of the housing.

The duct acts as confinement for the air inside the housing and can take the shape of a tube or channel formed as part of the housing or as a separate part connected to an opening in the housing. It can be a single straight or curved duct. The duct can be connected to more than one opening in the housing and can for example terminate at two ends with openings in the housing. The duct can further branch into several ducts or cavities, in which an actuator or a sensor may be located.

The opening itself can be a dedicated opening thus exclusively connecting the chemical sensor to the outside. However, given that the manufacturers of portable electronic devices strive to maintain the housing as a good protection against humidity and water, it is seen as advantageous that the opening is shared with at least one further component of the portable device requiring a similar connection to the exterior, such as a loudspeaker, a microphone or a camera. The opening can further be protected by a grill or a membrane to prevent bigger particles or unwanted components of the air from entering or blocking the duct.

The chemical sensor may be understood as a sensor device for detecting one or even more properties of one or more analytes. It is preferably based on one of the following measurement principles:
The sensor can be based on a chemomechanical principle, in which a chemical reaction is transformed into a surface acoustic wave, or into a cantilever resonance, for example. Alternatively, there may be thermal sensing concepts applied, e.g. by making use of pellistors which may serve as a catalytic thermal sensor in which heat is generated during combustion. Alternatively, the chemical sensor may rely on optical detection, such as in form of a microspectrometer, or an NDIR, or may make use of electrochemical reactions such as being enabled by solid state electrolytes in combination with voltammetric, potentiometric, or conductometric measurement principles. Chemiresistors may also be used, such as conducting and carbon-loaded polymers, preferably in a low-temperature arena, or, more preferably, metal-oxide sensors such as tin oxide, tungsten oxide, gallium oxide, indium oxide, zinc oxide, which preferably may be applied in a high temperature environment. ISFET (ion-selective FET) may also be used, as well as chemocapacitors wherein it is preferred to use a polymer as active material.

The sensor includes the sensor material, preferably in form of a layer, also denoted as receptor layer, to which an analyte may bond to and as such modify an electrical property of the sensor material such as its electrical conductance, which principle preferably is applied in metal oxide chemical sensors, or an optical property such as its transmission rate. It can also include a plurality of different sensors or an array of similar sensors. In such a sensor array, each sensor cell may provide a layer of a material exhibiting different absorption characteristics such that each cell of the sensor array may specifically be sensitive to a different analyte and as such may enable the portable electronic device to detect the presence or absence or concentration of such analyte.

The actuator linked to the duct can be a device capable of accelerating or stopping the air within the duct at least locally, i.e. close to the sensitive surface of the sensor. The driving force of the actuator is selected from a group including electro-acoustical or vibration, for example piezoelectric. Due to the space constraints in most portable electronic devices, it is preferred to make use of an actuator, which is not exclusively dedicated to the operation in conjunction with the chemical sensor but instead has at least a dual function providing a force used for other components of the portable device.

The above and other aspects of the present invention together with further advantageous embodiments and applications of the invention are described in further details in the following description and figures.

### BRIEF DESCRIPTION OF THE FIGURES

FIG. 1A is a perspective view of a portable electronic device;
FIG. 1B is a schematic view into part of the housing of the device of Fig. 1A;
FIGs. 2 illustrate examples (not claimed) using mechanical actuators;
FIGs. 3 illustrate examples in accordance with the present invention using electro-acoustic transducers or vibrators as actuators;
FIGs. 4 illustrate examples (not claimed) using heat sources as actuators;
FIG. 5 illustrates an example in accordance with the present invention using a combination of an actuator and a flow conditioning element; and
FIG. 6 illustrates a control scheme in accordance with an example of the present invention.

### DETAILED DESCRIPTION

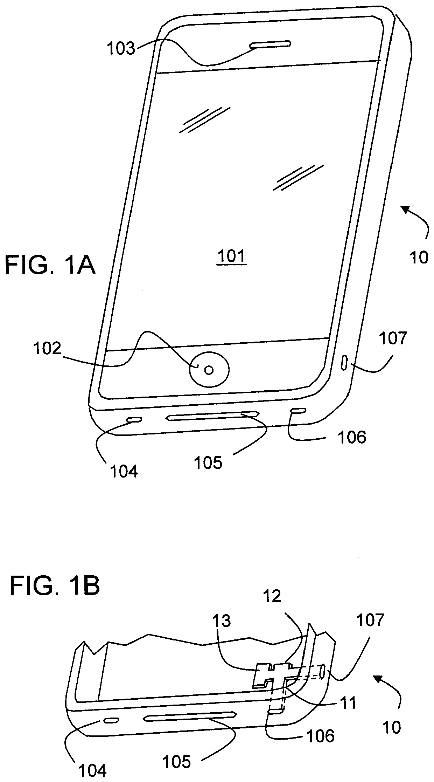

The device of FIG. 1A is a portable electronic device such as a mobile phone. The housing 10 of the mobile phone includes a front side with a screen 101 and elements like buttons 102 to let a user interact with the phone. Also shown on the front side is an opening 103 for a loudspeaker. Further openings 104,105 are located at a lower side wall of the housing 10. It is well known to mount components like microphones and loudspeakers behind such openings.

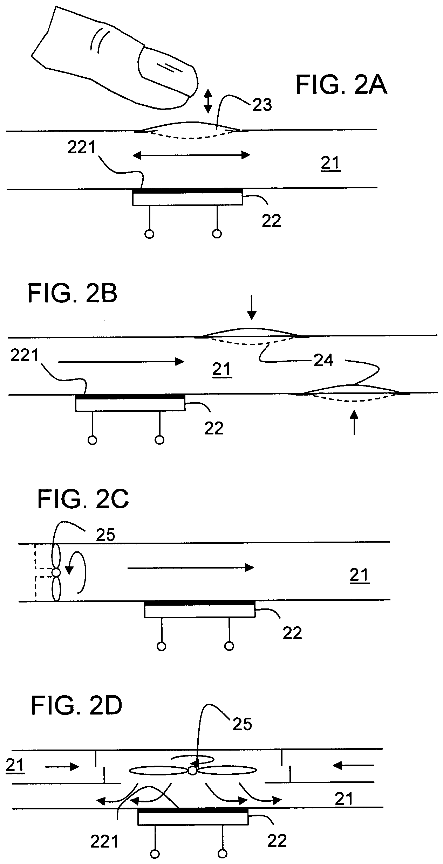

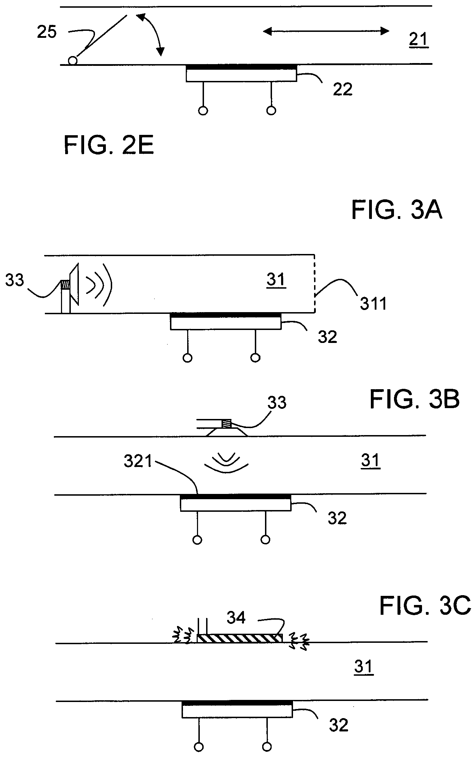

Another opening 106 is located at the lower side wall. As shown in FIG. 1B the opening 106 is linked to a tubular duct 11 passing through the interior of the housing. Whilst one opening is sufficient for an exchange of air between the interior of the housing and the exterior, in the example shown the duct exits the housing through another opening 107 at the right side wall of the housing 10. A chemical sensor 12 and an actuator 13 are both mounted along the duct 11 such that the actuator can influence the air movements in the duct and a sensitive area of the sensor is exposed to the air moving in the duct. The actual size and shape of the duct 11 depends on the volume available and the nature of the chemical sensor 12 and the actuator 13 and can vary to a large extent, as shown in further details in the following description of examples schematically illustrated in FIGs. 2-5 below.

In the example the chemical sensor is a gas sensor using a metal-oxide layer mounted onto and integrated with a CMOS substrate. The metal-oxide used can be tin oxide, tungsten oxide, gallium oxide, indium oxide, or zinc oxide. For particular embodiments as described in further details below the sensor can also include a micro electro-mechanical system or MEMS type heat source integrated within the sensor.

In the first series of examples illustrated in FIGs. 2 (not claimed) the actuator 23 provides a mechanical force driving the air flow through the duct 21.

For example in FIG. 2A there is shown the schematic view of a section of a duct 21 located within the housing of a portable electronic device. A chemical sensor 22 is mounted such that its sensitive surface 221 is exposed to the air in the duct 21. An air flow is generated along the duct 21 and hence along the surface 221 of the sensor 22 by means of manually operated fan or pump 23. The fan 23 is shaped as a part of the housing, which is designed to be pushed inwards and biased to return to its original shape when released. The part of the housing thus forms a simple pump or fan moving air across the surface 221 of the sensor 22.

The example of FIG. 2A can be automated using an actuator modulating the size of the duct locally. Using a suitable design as indicated in FIG. 2B, the modulation of the duct wall can create a pumping action within the duct similar to the known principle of a peristaltic pump 24.

In the example of FIG. 2C the mechanical force to drive the air flow is provided by a small fan or ventilator 25. Such fans are commercially available in sizes down to 8mm x 8mm with a height of 3mm and have been developed for cooling parts of mobile phones. In a variant of the fan-assisted air flow as shown in FIG. 2D, the fan 25 is mounted over the surface 221 of the sensor 22 and the duct 21 is folded such that air can flow off the surface in at least one direction.

In the example of FIG. 2E, the fan 25 is a simple flap moved essentially between two positions. The flap can be for example a thin metal leaf mounted on a hinge and moved between two positions by using for example alternatingly activated magnets or the like or in a more uncontrolled but energy-efficient manner by exploiting the movement or shaking of the portable device during normal usage.

In the second series of examples illustrated in FIGs. 3 the actuator is selected from transducers which are typically already present in portable electronic devices for other processes and purposes.

In the example of FIG. 3A, the duct 31 has a protective grill 311 at the opening to the exterior of the housing. The sensor 32 is located in the duct at a location between the grill 311 and a loudspeaker 33. The loudspeaker is a loudspeaker used for general sound reproduction in the portable device. The duct itself can be a duct directly connecting the loudspeaker to the exterior or a duct only acoustically coupled to the loudspeaker such as a bass reflex horn. It can be further advantageous to drive the general purpose loudspeaker 33 with a specific signal designed to increase the movement of air over the surface of the sensor. For example, a signal can be used which causes the emission of a sequence or wave train of very low frequency or bass sounds. It is also possible to enhance the effect of an acoustic signal by exploiting resonance frequencies such as the resonance frequencies of the air column in the duct 31 and drive the loudspeaker or any other wave source at or close to such a resonance frequency.

In the variant of FIG. 3B, the loudspeaker 33 is not located at one terminal of the duct 31 but a position opposite the sensitive surface 321 of the sensor 32.

In FIG. 3C, a transducer 34 conventionally applied for generating vibration alarms of the portable device is used to create an increased exchange of air at the location of the sensor 32. These transducers are known per se and can be built for example from piezoelectric elements.

In the examples above the transducer is placed in close proximity of the chemical sensor. However, as long as the motion of the actuator is transferred to the air in the duct, such a co-location of sensor and actuator is not required and the position of the actuator can be chosen more freely within the housing to make better use of the available space within the housing of the portable device.

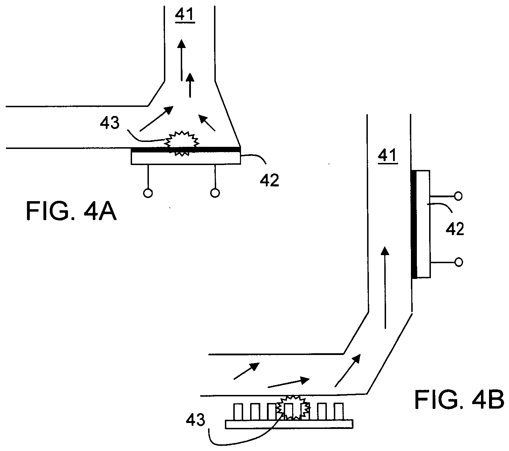

In the third series of examples illustrated in FIGs. 4 (not claimed) convection or thermal expansion caused by a heat source is used to increase the exchange of air in the duct over the surface of the chemical sensor. In the convection process a heat source generates an air flow in the duct with the sensor. In the example of FIG. 4A, the heat source 43 is part of the chemical sensor 42. Such a heat source on a chip is known per se for some type of sensors and often referred to as "hot-plate". The principles and further details concerning such hot-plates can be found for example in the United States patent no. 5464966 or other documents. Typically these hot-plates are required for the proper working of certain sensors such as for example the metal-oxide sensors.

The principles of these MEMS-type hot-plates manufactured using CMOS or other semiconductor fabrication methods can be applied to or used in conjunction with any chemical sensor in order to provide a heat source for convection with the duct 41 inside the portable device.

While for a typical metal-oxide sensor the heat source is necessarily located close to the actual sensor, in general the heat source for generating convection can be placed anywhere along the duct or close to it. The heat source is thus not necessarily a part of the sensor but can be a dedicated heat source using for example resistance heating, electro-thermal or Peltier effects or a MEMS-type hot plate on a dedicated chip or substrate. Such a dedicated heat source can be placed at any appropriate location along the duct for optimized convection.

The convection process can also be driven using excess heat from any of the standard components of the portable device. It is known that the processors used in such devices generate excess heat which needs to be dissipated to the exterior to avoid overheating of the device. The process can be exploited to carry excess heat through the duct 41 as shown in FIG. 4B. The component 43 generating the excess heat can be either positioned close to the duct 41 or linked to it via heat conductors such as heat pipes.

The convection over the sensor 42 can be further improved if at least part of the duct 41 is oriented upwards so as to provide an effect similar to a chimney. Thus the heat source 43 can be controlled using position information as provided by other components of the portable device, for example by accelerometers or gyroscopes. With such a control, the heat source is activated when the orientation sensor signals a suitable orientation of the portable device and, hence, of the duct 41.

Alternatively or in addition, the measuring process itself can be controlled using orientation information. For example it is possible to activate the chemical sensor for a (valid) reading or measurement only in predefined positions or orientations.

It is also possible to use the thermal expansion of air over the heat source as driving force to exchange air in the vicinity of the sensor. In such an application the heat source is best operated in an AC or a pulsating mode such that the periods of expansion of the air volume are followed by periods of contraction, effectively pumping new air into the volume above the sensor.

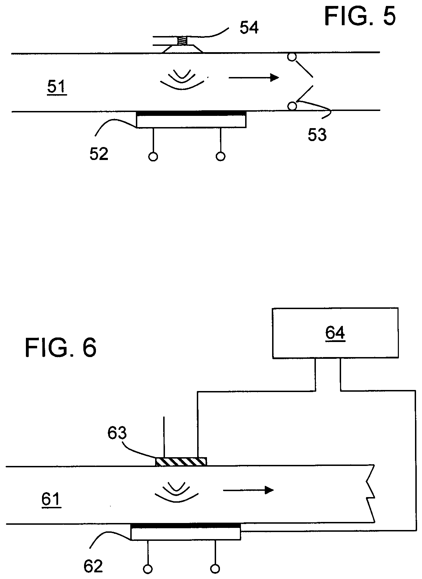

It is further possible to use two or more of the actuators as for example described above in combination to enhance the air flow at the location of the chemical sensor 52. In FIG. 5 there is shown a combination of a one-way air valve 53 in combination with a loudspeaker 54. The one-way valve 53 acts in analogy to a rectifier in an electric circuit and is thus used to transform a periodic movement or more random movement of air in the duct 51 into a directed flow. Other flow-shaping elements in the duct, such as constrictions, vanes and the like, designed to introduce asymmetrical flow conditions or a more directional flow can be used in place of the unidirectional valve 53.

As already mentioned above in connection with the orientation of the phone, the operation of the actuator and/or sensor can be controlled depending on a state of the portable device in general such as orientation, battery charge status, accidental blockage of the openings, and other states, which influence the performance of actuator and/or sensor. In these cases the control can initiate, interrupt, and end the operation of the sensor and/or actuator when the status of the portable device is not suited or ready for a satisfactory measurement.

In the example of FIG. 6, the transducer 63 and the chemical sensor 62 are linked using a control system 64, which determines beginning and the end of a period of forced air exchange in the duct 61 across the surface of the sensor 62. The control system, which is conveniently part of a general processing unit within the portable device, is also used to synchronize this period with the period of the actual measurement, during which the sensor performs an analysis of the air. The period of measurement best follows after the end of the period of forced air exchange to avoid a perturbation of the measurement by the actuator and the forced air flow.

While there are shown and described presently preferred embodiments of the invention, it is to be distinctly understood that the invention is not limited thereto but may be otherwise variously embodied and practised within the scope of the following claims.

## Claims

1. Portable electronic device, comprising
- a housing (10)
- a chemical sensor (12,22,32,42,52,62) arranged inside the housing (10), which chemical sensor (12,22,32,42 52,62) is adapted to measure a property of at least one analyte,
a duct (11, 21, 31, 41, 51, 61) germinating at an opening (196,107) in the housing (10) for exposing the chemical sensor to the fluid to be analyzed, and an actuator (13) to create an air flow within the duct, **characterized in**
**that** the actuator (13) comprises a vibratory actuator (34) applied for generating vibration alarms of the portable electronic device, or comprises an electro-acoustic transducer (33) in form of a loudspeaker used for general sound reproduction in the portable electronic device generating a sound wave in the duct (31).

2. The portable electronic device according to claim 1, wherein the actuator (13) comprises a fan (23,25) .

3. The portable electronic device according to claim 1, wherein the actuator (13) comprises a pump (24) within the duct (31).

4. The portable electronic device according to claim 1, wherein the actuator (13) comprises a heat source (43) to generate a flow through convection or thermal expansion of the air in the duct (41).

5. The portable electronic device according to any of the preceding claims, further comprising a flow conditioning element (53) generating in operation a preferential direction for flow in the duct (51).

6. The portable electronic device according to claim 5, wherein the flow conditioning element is a one-way valve (53).

7. The portable electronic device according to any of the preceding claims, wherein the duct comprises a cover sufficiently permeable to allow analyte to pass through to the chemical sensor.

8. The portable electronic device according to any of the preceding claims, wherein the duct (11) comprises a second opening (107) in the housing (10) such that air entering the duct (11) through one of the openings (106) can exit the duct through the other opening (107).

9. The portable electronic device according to any of the preceding claims, wherein the actuator (63) and/or sensor (62) are connected to a control system (64) for activating or deactivating the actuator and/or sensor, wherein the control system is adapted to register at least one state of the portable device and controls the actuator and/or sensor depending on the state registered.

10. The portable electronic device according to claim 9, wherein the state is selected from a group including the spatial orientation of the portable device, battery charge, prior or actual time periods of air exchange in or volume of air flow through the duct (61).

11. The portable electronic device according to any of the preceding claims, wherein the chemical sensor (12,22,32,42 52,62) comprises a metal-oxide sensing material.

12. The portable electronic device according to any of the preceding claims, wherein the chemical sensor (12,22,32,42,52,62) comprises a metal-oxide sensing material mounted and electrically connected to a substrate including CMOS circuitry.

13. The portable electronic device according to any of the preceding claims, wherein the chemical sensor (12,22,32,42,52,62) and the actuator (13,63) are connected to a common control system (64) programmed to act on a request for a measurement by first initiating the actuator (13,63) and after a period of forced flow in the duct (11,61) deactivating the actuator (13,63) to end the forced flow in the duct before initiating the chemical sensor to perform a measurement.

14. The portable electronic device according to any of the precedings claims, being selected from a group comprising:
a mobile phone,
a handheld computer,
an electronic reader,
a tablet computer,
a game controller,
a pointing device,
a photo or a video camera,
a digital music player,
a wrist watch,
a key fob,
a head set, and a computer peripheral.

## Patentansprüche

1. Tragbare elektronische Vorrichtung umfassend
- ein Gehäuse (10)
- einen chemischen Sensor (12, 22, 32, 42, 52, 62), angeordnet im Gehäuse (10), wobei der chemische Sensor (12, 22, 32, 42, 52, 62) zur Messung einer Eigenschaft mindestens eines Analyts ausgestaltet ist,
eine Leitung (11, 21, 31, 41, 51, 61) die an einer Öffnung (196, 107) im Gehäuse endet, um den chemischen Sensor einem zu analysierenden Fluid auszusetzen, und einen Antrieb (13) zur Erzeugung eines Luftflusses in der Leitung, **dadurch gekennzeichnet, dass**
der Antrieb (13) einen Vibrationsantrieb (34) umfasst, der zur Generierung von Vibrationsalarmen der tragbaren elektronischen Vorrichtung angewendet wird, oder einen elektroakustischen Transducer (33) umfasst, in Form eines Lautsprechers, der für eine generelle Tonwiedergabe in der tragbaren elektronischen Vorrichtung, der eine akustische Welle in der Leitung generiert, verwendet wird.

2. Die tragbare elektronische Vorrichtung nach Anspruch 1, wobei der Antrieb (13) einen Ventilator (23, 25) umfasst.

3. Die tragbare elektronische Vorrichtung nach Anspruch 1, wobei der Antrieb (13) eine Pumpe (24) in der Leitung (31) umfasst.

4. Die tragbare elektronische Vorrichtung nach Anspruch 1, wobei der Antrieb (13) eine Wärmequelle (43) zur Generierung eines Flusses durch Konvektion oder thermische Expansion der Luft in der Leitung (41) umfasst.

5. Die tragbare elektronische Vorrichtung nach einem der vorangehenden Ansprüche, weiter umfassend ein Flusskonditionierungselement (53), das im Betrieb eine bevorzugte Flussrichtung in der Leitung (51) generiert.

6. Die tragbare elektronische Vorrichtung nach Anspruch 5, wobei das Flusskonditionierungselement ein Einwegventil (53) ist.

7. Die tragbare elektronische Vorrichtung nach einem der vorangehenden Ansprüche, wobei die Leitung eine Abdeckung umfasst, die ausreichend durchlässig ist um dem Analyten zu erlauben, durch den chemischen Sensor zu passen.

8. Die tragbare elektronische Vorrichtung nach einem der vorangehenden Ansprüche, wobei die Leitung (11) eine zweite Öffnung (107) im Gehäuse (10) umfasst, so dass die Luft, die in die Leitung (11) durch eine der Öffnungen (106) eintritt, durch die andere Öffnung (107) aus der Leitung austreten kann.

9. Die tragbare elektronische Vorrichtung nach einem der vorangehenden Ansprüche, wobei der Antrieb (63) und/oder der Sensor (62) mit einem Steuerungssystem (64) verbunden ist/sind, um den Antrieb und/oder den Sensor zu aktiveren und zu deaktivieren, wobei das Steuerungssystem zur Registrierung mindestens eines Zustands der tragbaren Vorrichtung ausgestaltet ist und den Antrieb und/oder den Sensor in Abhängigkeit vom registrierten Zustand steuert.

10. Die tragbare elektronische Vorrichtung nach Anspruch 9, wobei der Zustand aus einer Gruppe umfassend die örtliche Ausrichtung der tragbaren Vorrichtung, den Batterieladestand, vorherige oder aktuelle Zeitintervalle von Luftaustausch in der oder das Volumen des Luftflusses durch die Leitung (61) ausgewählt ist.

11. Die tragbare elektronische Vorrichtung nach einem der vorangehenden Ansprüche, wobei der chemische Sensor (12, 22, 32, 42, 52, 62) ein Metalloxid-empfindliches Material umfasst.

12. Die tragbare elektronische Vorrichtung nach einem der vorangehenden Ansprüche, wobei der chemische Sensor (12, 22 32, 42, 52, 62) ein Metalloxid-empfindliches Material umfasst, das auf einem Substrat, das CMOS-Schaltungen einschliesst, aufgebaut und mit diesem verbunden ist.

13. Die tragbare elektronische Vorrichtung nach einem der vorangehenden Ansprüche, wobei der chemische Sensor (12, 22 32, 42, 52, 62) und der Antrieb (13, 63) mit einem gemeinsamen Steuerungssystem (64) verbunden sind, das programmiert ist, um auf eine Messungsanfrage zu reagieren, indem es zunächst den Antrieb (13, 63) startet und nach einem Intervall von erzwungenem Fluss in der Leitung (11, 61) den Antrieb (13, 63) abschaltet, bevor es den chemischen Sensor zur Durchführung einer Messung startet.

14. Die tragbare elektronische Vorrichtung nach einem der vorangehenden Ansprüche, ausgewählt aus einer Gruppe umfassend:
ein Mobiltelefon,
einen Taschencomputer,
ein elektronisches Lesegerät,
ein Tablett,
eine Spielkonsole,
ein Zeigegerät,
eine Foto- oder eine Videokamera,
ein digitales Abspielgerät,
eine Armbanduhr,
einen Schlüsselanhänger,
ein Headset,
und eine Computerperipherie.

## Revendications

1. Dispositif électronique portable comprenant
- une carcasse (10)
- un capteur chimique (12,22,32,42,52,62) arrangé à l'intérieur de la carcasse (10), le capteur chimique (12,22,32,42,52,62) étant adapté à mesurer une propriété d'au moins un analyte,
- un tuyau (11, 21, 31, 41, 51, 61) qui finit à une ouverture (196, 107) dans la carcasse (10) pour exposer le capteur chimique au fluide à analyser, et un entrainement (13) pour créer un écoulement d'air dans le tuyau, **caractérisé en ce que**
l'entrainement (13) comprend un entrainement vibrant (34) appliqué pour créer des alarmes vibratoires du dispositif électronique portable, ou comprend un transducteur électrochimique (33) en forme d'un haut-parleur utilisé pour une reproduction de son générale dans le dispositif électronique portable en générant une onde sonore dans le tuyau (31).

2. Le dispositif électronique portable selon la revendication 1, l'entrainement (13) comprenant un ventilateur (23, 25).

3. Le dispositif électronique portable selon la revendication 1, l'entrainement (13) comprenant une pompe (24) dans le tuyau (31).

4. Le dispositif électronique portable selon la revendication 1, l'entrainement (13) comprenant une source de chaleur (43) pour générer un écoulement par convection ou expansion thermique de l'air dans le tuyau (41).

5. Le dispositif électronique portable selon l'une des revendications précédentes, comprenant en outre un élément de conditionnement d'air (53) qui génère en opération une direction de préférence pour l'écoulement dans le tuyau (51).

6. Le dispositif électronique portable selon la revendication 5, l'élément de conditionnement d'écoulement étant une soupape unidirectionnelle (53).

7. Le dispositif électronique portable selon l'une des revendications précédentes, le tuyau comprenant une couverture suffisamment perméable pour permettre le passage de l'analyte à travers le capteur chimique.

8. Le dispositif électronique portable selon l'une des revendications précédentes, le tuyau (11) comprenant une deuxième ouverture (107) dans la carcasse (10) de sorte que l'air qui entre dans le tuyau (11) à travers une des ouvertures (106) puisse sortir du tuyau à travers l'autre ouverture (107).

9. Le dispositif électronique portable selon l'une des revendications précédentes, l'entrainement (63) et/ou le capteur (62) étant connecté(s) à un système de commande (64) pour activer ou désactiver l'entrainement et/ou le capteur, le système de commande étant adapté à enregistrer au moins un état du dispositif portable et commande l'entrainement et/ou le capteur dépendant de l'état enregistré.

10. Le dispositif électronique portable selon la revendication 9, l'état étant sélectionné d'un groupe incluant l'orientation spatiale du dispositif portable, le chargement de la batterie, des périodes de temps antérieures ou actuelles d'une échange d'air dans ou du volume d'écoulement d'air à travers le tuyau (61).

11. Le dispositif électronique portable selon l'une des revendications précédentes, le capteur chimique (12, 22, 32, 42, 52, 62) comprenant un matériau sensible à un oxyde de métal.

12. Le dispositif électronique portable selon l'une des revendications précédentes, le capteur chimique (12, 22, 32, 42, 52, 62) comprenant un matériau sensible à un oxyde de métal monté et connecté électriquement à un substrat incluant des circuits CMOS.

13. Le dispositif électronique portable selon l'une des revendications précédentes, le capteur chimique (12, 22, 32, 42, 52, 62) et l'entrainement (13, 63) étant connectés à un système de commande commun (64) programmé à actionner basé sur une demande pour une mesure en premièrement démarrant l'entrainement (13, 63) et ensuite, après une période d'écoulement forcé dans le tuyau (11, 61), en désactivant l'entrainement (13, 63) afin de terminer l'écoulement forcé dans le tuyau avant démarrer le capteur chimique pour effectuer une mesure.

14. Le dispositif électronique portable selon l'une des revendications précédentes, étant sélectionné du groupe comprenant:
un téléphone portable,
un ordinateur portable,
un lecteur électronique,
une tablette,
un contrôleur de jeu,
un dispositif de pointage,
une caméra photo ou vidéo,
un lecteur de musique digital,
une montre à bracelet,
un porte-clés,
une casque-micro,
et une périphérie d'ordinateur.
